# EUROPEAN PATENT SPECIFICATION

(11) **EP 2 152 313 B1**
(45) Date of publication and mention of the grant of the patent: **03.09.2014**
(21) Application number: 08741851.3
(22) Date of filing: 21.04.2008
(51) Int. Cl.: A61K 47/18, A24B 15/16, A24B 15/30, A61K 31/465, A61K 9/08, A61K 9/20, A61K 9/48, A61K 9/68, A61P 25/34, A23L 1/305, A23G 3/44, A23G 4/14, A61K 9/00

(54) **ORAL NICOTINE FORMULATION BUFFERED WITH AMINO ACID**
MIT AMINOSÄURE GEPUFFERTE ORALE NIKOTIN-FORMULIERUNG
FORMULATION NICOTINIQUE ORALE TAMPONNÉE AUX ACIDES AMINÉS

(30) Priority: 16.05.2007 SE 0701178
(43) Date of publication of application: 17.02.2010
(73) Proprietor: McNeil AB, 25109 Helsingborg (SE)
(72) Inventor: ANDERSSON, Sven-Börje, SE-260 35 Odäkra (SE); BERGENGREN, Gunnar, SE-25223 Helsingborg (SE); BOSSON, Bengt, SE-256 55 Helsingborg (SE); HUGERTH, Andreas, SE-23733 Bjärred (SE); NICKLASSON, Fredrik, SE-23731 Bjärred (SE); OLSSON, Roland, SE-253 76 Helsingborg (SE)
(74) Representative: Kirsch, Susan Edith
(86) International application number: PCT/SE2008/000277
(87) International publication number: WO 2008/140371

(56) References cited:
- EP-A1- 1 304 048
- WO-A1-02/102357
- WO-A1-02/102357
- WO-A1-03/055486
- WO-A2-2005/023227
- BECKETT A H ET AL: "Buccal absorption of basic drugs and its application as an in vivo model of passive drug transfer through lipid membranes", JOURNAL OF PHARMACY AND PHARMACOLOGY, JOHN WILEY & SONS LTD, LONDON; GB, vol. 19, 1 December 1967 (1967-12-01), pages 31S-41S, XP009142973, ISSN: 0022-3573
- OGAWA T. ET AL.: 'Screening of Bitterness-Suppressing Agents for Quinine: The Use of Molecularly Imprinted Polymers' JOURNAL OF PHARMACEUTICAL SCIENCES vol. 94, no. 2, February 2005, pages 353 - 362, XP009068797
- OGAWA T. ET AL.: 'The Combination Effect of L-Arginine and NaCl on Bitterness Supression of molecularly imprinted polymers' CHEM. PHARM. BULL. vol. 52, no. 2, 2004, pages 172 - 177, XP003023513

## Description

### Technical Field

This invention relates to nicotine-containing pharmaceutical formulations for intraoral delivery of nicotine to a subject. The formulations comprise one or more amino acids as buffering agents. Of specific interest are endogenous amino acids. Also contemplated are a method and a system for delivering nicotine as well as use and production of said formulations.

### Background of the Invention

Tobacco dependence and reduction thereof is a desirable goal. In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief addictive ingredient in the tobacco used in cigarettes, cigars, snuff and the like. Nicotine is also an addictive drug, and smokers characteristically display a strong tendency to relapse after having successfully stopped smoking for a time. Nicotine is the world's second most used drug, after caffeine from coffee and tea.

The main problem with tobacco smoking is its enormous implications on health. It is estimated that smoking related diseases cause some 3 - 4 million deaths per year. According to Centers for Disease Control and Prevention, cigarette smoking among adults - United States, 1995. MMWR 1997; 46:1217 - 1220 around 500,000 persons in USA die each year as a result of tobacco use. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug. The incidence of smoking is still rising in many countries, especially in less developed countries.

The most advantageous thing a heavy smoker can do is to stop smoking completely or at least to reduce his/her smoking. Experience shows, however, that most smokers find this extremely difficult since, mostly, tobacco smoking results in a dependence disorder or craving. The World Health Organization ("WHO") has in its International Classification of Disorders a diagnosis called Tobacco Dependence. Others like the American Psychiatric Association call the addiction Nicotine Dependence. It is generally accepted that these difficulties to stop smoking result from the fact that those heavy smokers are dependent on nicotine. The most important risk factors related to health are, however, substances that are formed during the combustion of tobacco, such as carcinogenic tar products, carbon monoxide, N-nitrosamines, aldehydes, and hydrocyanic acid.

### Effects of nicotine

Nicotine is an addictive poisonous alkaloid C₅H₄NC₄H₇NCH₃, derived from the tobacco plant. Nicotine is also used as an insecticide. The administration of nicotine (for example, in the form of smoking a cigarette, cigar or pipe) can give a pleasurable feeling to the smoker. However, smoking has health hazards and it is, therefore, desirable to formulate an alternative way of administering nicotine in a pleasurable and harmless manner that can be used to facilitate withdrawal from smoking and/or used as a replacement for smoking.

When smoking a cigarette, nicotine is quickly absorbed into the smoker's blood and reaches the brain within around ten seconds after inhalation. The quick uptake of nicotine gives the consumer a rapid satisfaction, or kick. The satisfaction usually lasts during the smoking time of the cigarette and for a period of time thereafter. The poisonous, toxic, carcinogenic, and addictive nature of smoking has provided strong motivation to develop methods, compositions and devices, which can be used to break the habit of smoking cigarettes.

### Nicotine replacement products

One way to reduce smoking is to provide nicotine in a form or manner other than by smoking and some products have been developed to fulfil this need. Nicotine containing formulations are currently the dominating treatments for tobacco dependence.

The successes in achieving reduction in the incidence of smoking have been relatively poor using presently known products. The present state of the art involves both behavioural approaches and pharmacological approaches. More than 80 % of the tobacco smokers who initially quit smoking after using some behavioural or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market. Several methods and means have been described for diminishing the desire of a subject to use tobacco, which comprises the step of administering to the subject nicotine or a derivative thereof as described in e g U.S. Patent Number 5,810,018 (oral nicotine-containing spray), U.S. Patent Number 5,939,100 (nicotine- containing micro spheres) and U.S. Patent Number 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Ways of administrating nicotine by way of delivering directly into the nasal cavity by spraying is known from U.S. Patent Number 4,579,858, DE 32 41 437 and WO93/12764. There may be local nasal irritation, however, with use of nasal nicotine formulations. The difficulty in administration also results in unpredictability of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in U.S. Patent Number 5,167,242. Said means and methods address the problems associated with addiction to nicotine.

One successful product that is used as a smoking substitute and/or as a smoking cessation aid and which is based on nicotine, is the chewing gum Nicorette^{®}. This product was one of the first nicotine replacement forms that was approved by the Food and Drug Administration (FDA) and is still one of the most used nicotine replacement products. Nicorette^{®} chewing gum has been on the market in about 60 countries for several years. In this chewing gum the nicotine is present in the form of a complex with an insoluble cation-exchanger (polacrilex) that is dispersed in a gum base. The nicotine is slowly released from the gum due to chewing and will reach similar plasma levels as when smoking a cigarette after about 30 minutes depending on the chewing technique, i e slow or active. Patents related to this product are e g U.S. Patent Number 3,877,468, U.S. Patent Number 3,901,248 and U.S. Patent Number 3,845,217.

### Prior art and problems thereof

WO 02/102357 discloses a coated nicotine-containing chewing gum. This gum provides improved transmucousal absorption of nicotine in the oral cavity. Thereby is achieved more of a cigarette-like sense of satisfaction and a more rapid reduction of the urge to smoke. Most buffers proposed in WO 02/102357 possess off-notes, however, and one or more flavouring agents need be added to the gum in order to cover the off note taste. Certain salts of glycine are mentioned in a list of buffers without any mentioning of whether these salts have any disagreeable taste or not.

WO 2005/023227 discloses nicotine-containing compositions wherein nicotine is absorbed into and/or onto cellulose of non-seed organism origin, especially from algae, bacteria and/or fungi. As with WO 02/102357 also most buffers proposed in WO 2005/023227 possess off-notes. Certain salts of glycine are mentioned in a list of buffers without any mentioning of whether these salts have any disagreeable taste or not.

Oqawa Tazuko et al.:"Screening of bitterness-suppressing agents for quinine: The use of molecularly imprinted polymers"; Journal of Pharmaceutical Sciences, Vol. 94, No. 2 (Feb 2005), 353 -362, assumes that a few amino acids may suppress the bitterness of quinine, while most amino acids do not suppress such bitterness. Anyhow, Oqawa et al do not disclose any utility for amino acids as buffering agents in nicotine-containing formulations. Quinine and nicotine are chemically and pharmacologically very different, which means that teachings on quinine cannot as such be applied on nicotine.

WO 03/055486 seeks to provide formulations with fast satisfaction of nicotine craving without smoking.

Problems with off-notes from buffers may arise with all other nicotine-containing pharmaceutical formulations for oral delivery, such as mouth sprays, chewable tablets, tablets, lozenges, chewing gums, capsules, lipid-filled micro gels, oral films, and different candy-type formulations.

Thus, there is a need to ameliorate nicotine-containing pharmaceutical formulations for oral delivery in order to avoid off-notes from the buffers used. Many of the buffering compounds disclosed as novel and inventive in the present application are especially useful as they are endogenous amino acids, i e they are already present in the human body.

To date nothing has been disclosed on the utility of amino acids as buffers in nicotine-containing pharmaceutical formulations. US 5,733,572 discloses gas filled micro-spheres, which, as stated in a long and non-substantiated laundry list, may further comprise nicotine and certain amino acids, the latter though not for buffering purposes, but for achieving a depot action effect.

### Summary of the Invention

When formulating a medical product intended to dissolve in the oral cavity the organoleptic characteristics are essential. Besides, in many cases there is a need to obtain optimal pH in the oral cavity to be able to achieve sufficiently rapid uptake of the active ingredient. By using a buffering agent in the product said pH can be adjusted. However, the number of pharmaceutically appropriate buffering agents is limited and some of the most commonly used buffering agents possess distinct off-notes. Therefore, one or more flavoring agents are usually added to the formulation to cover these off-notes. Moreover, flavoring agents are also used in the formulation to accomplish a product with pleasant taste. The possibility of using a buffering agent with no or comparatively mild off-taste, facilitates the formulation work and reduces the complexity of the flavoring process. Many of the amino acid type of buffering agents possess no unpleasant intrinsic taste and consequently, the use of such excipients in nicotine-containing products for oral uptake has been found to be beneficial by the present inventors.

Another important criterion when choosing buffering agents in nicotine-containing formulations is the toxicity. Many of the common amino acids can be classified as harmless since they are present in large amounts, several grams per day, in common nutrition.

Other advantages in using amino acids as buffers in nicotine-containing formulations encompass lack of unpleasant smell and that many of the amino acids of interest have monographs in both USP/NF and Ph.Eur and that many of them are found in the FDA-list of inactive ingredients.

In view of the foregoing disadvantages known in the art when trying to deliver nicotine to a subject so as to obtain a transmucousal uptake of nicotine in the oral cavity of the subject the present invention provides a new and improved product, systems and methods for obtaining a transmucousal uptake of nicotine in the oral cavity of the subject, while avoiding off-notes from the buffer used.

An object of the present invention is to provide an efficient and effective product, as well as methods and systems for uptake of nicotine in a subject and to avoid the disadvantages of such previously known products and methods.

The present invention provides for an oral formulation according to claim 1 comprising nicotine in any form, which is buffered with at least one amino acid and which comprises a pH-adjusting compound should the pH-adjusting capacity of said at least one amino acid be insufficient.

Also described is a method for delivering nicotine in any form to a subject comprising administering to a subject an oral formulation comprising nicotine in any form into the oral cavity of the subject and if needed allowing the nicotine in any form in the oral formulation to be released in the saliva in the oral cavity and absorbed into the systemic circulation of the subject as well as a method for producing said oral formulation. In a mouth spray the nicotine is directly available wherefore it need not be released as such in the saliva as said above. Hence the phrase "if needed" is inserted in the preceding sentence and in corresponding sentences below and in the claims.

The present invention further provides a use of the oral formulation described herein for obtaining reduction of the urge to smoke or use tobacco containing material and/or for providing a sense of smoking satisfaction without smoking, by the steps of replacing at least partly the tobacco containing material with the above said oral formulation, administering to a subject an oral formulation containing nicotine in any form into the oral cavity of the subject and if needed allowing the nicotine in any form of the oral formulation to be released in the saliva in the oral cavity and absorbed by the subject.

Furthermore, the present invention provides a system for delivering nicotine in any form to a subject, comprising said oral formulation and at least one other means for obtaining reduction of the urge to smoke or use of tobacco as well as a system for obtaining reduction of the urge to smoke or otherwise use tobacco and/or for providing a sense of smoking satisfaction without smoking, comprising an oral formulation as described above and at least one other method for obtaining reduction of the urge to smoke or otherwise use tobacco. Said system may be a system wherein the at least other method is selected from the group consisting of administration through mouth sprays, nasal sprays, transdermal patches, inhaling devices, lozenges, tablets and parenteral methods, subcutaneous methods, and transmucousal methods; or other use of tobacco.

By using an amino acid as the only buffer, or as the main buffer, in said oral formulation the aforesaid problem with off-notes from the buffers used is solved. Further, such a buffer can, as said supra, be regarded as adequate from a toxicity point of view.

### Detailed Description of the Invention

### Definitions

As used herein, the term "oral formulation" or similar intends to mean all formulations being suitable to be placed in the oral cavity for delivering nicotine essentially to the mucous membrane of the oral cavity.

The term "intraoral delivery" is herein intended to mean delivery into the systemic blood circulation by means of absorption of an active principle by any tissue of the oral cavity.

The term "complete reduction" or "complete" is herein intended to mean complete or substantially complete reduction.

The term "controlled release" is intended to mean a release of nicotine from an oral formulation in the oral cavity of the subject, whereby active sucking or other manipulation of the oral formulation is controlling the amount of nicotine released.

The term "slow release" is intended to mean that nicotine is released from the oral formulation upon sucking or other manipulation over a period of time for example, several minutes to an hour.

The term "unit formula" is intended to mean one oral formulation unit.

The term "transient" is intended to mean a non-permanent change, upon which the relevant state, e g biological or physiological state, after a certain period of time will return to its value or behaviour prior to said change.

The terms "buccal" and "buccally" are herein intended to pertain to all of or any part of the tissue of the oral cavity.

### Useful oral formulations

Most dosage forms intended for oral delivery of nicotine benefit from using selected amino acids as the only or main buffering agent. These formulations include e g mouth sprays, chewing gums, tablets, melt tablets, lozenges, hard boiled candies, chewy candies, gummies, capsules, oral films, and liquid as well as powder formulations for intraoral and pulmonary inhalation.

Particular formulations are mouth sprays. These are discreet dosage forms being useful for obtaining a rapid uptake of nicotine through the mucosa of the oral cavity. Mouth sprays may sprayed straight into the oral cavity or alternatively under the tongue. Below Example 3 discloses the manufacturing of a mouth spray according to the invention.

The amount of gum base in a chewing gum according to the invention is about 15 - 80 % by weight of the total gum core, and preferably about 40-80%. The amount of gum base employed for slow release of nicotine is usually in the higher ranges when nicotine is employed *per se* or when an absorbed form is used.

The gum base may be of any conventional nature known in the art. For example, it may comprise a gum base of natural or synthetic origin readily available from a commercial source. Natural gum bases include e g chicle, jelutong-, lechi de caspi-, soh-, siak-, katiau-, sorwa-, balata-, pendare-, malaya-, and peach gums, natural cautchouc and natural resins such as dammar and mastix. Synthetic gum bases are a mixture of:
- elastomers (for example polymers and/or masticating substances),
- plasticizers (for example resins, elastomers and/or solvents)
- fillers (for example texturizers and/or water-insoluble adjuvants),
- softeners (for example fats),
- emulsifiers,
- waxes,
- antioxidants,
- and anti-tacking agents (for example vinyl polymers and/or hydrophilic resins)

Other examples of gum bases are gums including agar, alginate, arabic gum, carob gum, carrageenan, ghatti gum, guar gum, karaya gum, pectin, tragacanth gum, locust beam gum, gellan gum and xanthan gum.

Examples of gelling agents comprise gum arabic, starch, gelatine, agar, and pectin.

When the nicotine in any form and the buffering agent or agents are incorporated in the chewing gum mass in accordance with the present invention, it is possible to employ a wide variety of chewing gum compositions and amounts of the chewing gum base.

Different chewing gum products may be composed depending on the consumers' preference and the purpose of use, in respect of the nicotine level, nicotine distribution and other additives.

Further below follow Examples on useful chewing gums, tablets, melt tablets, mouth sprays, soft capsules, hard boiled candies, oral films, gummies and chewy candies according to the present invention. On the basis of said Examples also other useful embodiments are envisageable.

### The buffering agent

Absorption of nicotine from the oral cavity to the systemic circulation is dependent on the pH of the saliva, pH of the blood plasma and the acid-base equilibrium of nicotine, which is about pKa = 7.8 at 37° C. Assuming a pH of the saliva of 6.8, only about 10% of the nicotine will be in the non-charged base form. Thus, in order to promote absorption of nicotine in a free base form, which is the form predominantly absorbed through the mucosa, the pH of the saliva must be increased. At a pH of 9.0 more than 90% of the nicotine will be in the free readily absorbable base form.

According to the invention, the oral formulation is buffered by use of substances, agents or other means, which at least partly comprise an amino acid, preferably an endogenous amino acid, and/or a salt thereof.

As said above many of the amino acid type of buffering agents possess no unpleasant intrinsic taste. Further, many of the common amino acids, especially the endogenous ones, can be classified as harmless from a toxicity point of view since they are present in large amounts, several grams per day, in common nutrition.

As least some of the below criteria should preferably be used when selecting amino acids useful as buffers in nicotine-containing formulations:
1) pKa in the interval 8,0 - 9,6 (as the system should buffer in the pH area above nicotine's pKa value at 25°C).
2) Solubility in water more than around 10 g/kg.
3) Useful from a toxicity point of view.
4) Preferably already used as buffer in pharmaceutical formulations devoid of nicotine.

The most useful amino acids are listed in below Table 1.

**Table 1 Especially useful amino acids.**

| **Compound** | **CAS Number** | **pKa value (in interval 8 - 9,6)** | **Solubility in Water, g/kg** |
|---|---|---|---|
| Arginine | 74-79-3 | 9,00 | 182,6^{a)} |
| Aspargine | 70-47-3 | 8,73 | 25,1 |
| Glutamic acid | 56-86-0 | 9,58 | 8,61^{a)b)} |
| Glutamine | 56-85-9 | 9,00 | 42 |
| Glycine* | 56-40-6 | 9,58 | 250,9 |
| Histidine | 71-00-1 | 9,09 | 43,5 |
| Isoleucine | 73-32-5 | 9,60 | 34,2 |
| Leucine | 61-90-5 | 9,58 | 22,0 |
| Lysine | 56-97-1 | 9,16 | Very soluble ^{a)b)} |
| Methionine | 63-68-3 | 9,08 | 56 |
| Phenylalanine | 63-91-2 | 9,09 | 27,9 |
| Serine | 56-45-1 | 9,05 | 50,2 |
| Threonine | 72-19-5 | 8,96 | 98,1 |
| Valine | 72-18-4 | 9,52 | 88,5 |
| Cysteic acid | 13100-82-8 | 8,70 | Very soluble |
| N-Glycylglycine | 556-50-3 | 8,10 | No information |
| Ornithine | 70-26-8 | 8,78 | Very soluble |

| | | | |
|---|---|---|---|
| * = Comparative a) reported as buffer in non-nicotine-containing pharmaceutical formulations. b) low or uncertain value on solubility in water. | | | |

The captioned data on the amino acids are taken from "Handbook of Chemistry and Physics", 85th edition; Table 7-1 ("20 standard amino acids that are the basic constituents of proteins") and Table 7-2 ("Amino acids and related compounds of biochemical importance").

The buffering is designed so as to achieve a transient buffering of the saliva of a subject at an elevated pH value during melting, disintegration or dissolution of the oral formulation. As the change is transient, the pH will return to its normal value after a certain period of time.

By employing said change, here an increase, in said pH of the saliva the transmucousal uptake of nicotine in the oral cavity is changed, e g increased compared to the nicotine uptake when the saliva is not buffered according to the invention. Also, since the transmucousal uptake of nicotine in the oral cavity according to the invention is faster than for nicotine not being buffered according to the invention, less nicotine will be swallowed to reach the gastrointestinal (G.I.) tract. The nicotine that reaches the G.I. tract will be subjected to first pass metabolism, which reduces the total amount of intact nicotine absorbed. This means that the bioavailability of nicotine that is not co-administered with a buffer will generally be lower than when administered together with a buffer.

Further embodiments of the invention includes oral dosage forms being buffered with an amino acid in combination with other buffers or pH-adjusting compounds, preferably selected from the group consisting of a carbonate including bicarbonate or sesquicarbonate, phosphate, glycero-phosphate or citrate of an alkali metal, such as potassium or sodium, or ammonium, and mixtures thereof.

Still further embodiments may encompass use of an amino acid together with different phosphate systems, such as trisodium phosphate, disodium hydrogen phosphate; and tripotassium phosphate, dipotassium hydrogen phosphate, and calcium hydroxide, sodium glycinate, trometamol and mixtures thereof.

Alkali metal carbonates and phosphates are preferred additional buffering agents or pH-adjusting compounds.

In order to increase the buffering capacity still further without correspondingly increasing the pH, one may in specific embodiments use a second or auxiliary buffering agent to the first buffering agent, such as e g sodium or potassium bicarbonate buffers. In addition a strong base, e g sodium hydroxide, may be added to the formulation in order to raise its basic property. Thereby should be strived to maintain a pleasant taste. The second or auxiliary buffering agent may be selected from the group consisting of alkali metal bicarbonates that are preferred for this purpose. Thus, further embodiments of the invention may comprise an amino acid and a mixture of an alkali metal carbonate or phosphate and alkali metal bicarbonate. Some useful mixture ratios are provided in the below Examples.

The amount of the buffering agent and optional pH-adjusting compounds in the oral formulation is preferably sufficient in the specific embodiments to transiently maintain the pH of the saliva in the oral cavity above 7, e g pH 7 - 10.

The nicotine may be administered in different forms, e g in different complexes or salts. The amount of buffer and optional pH-adjusting compounds required to achieve an increase in pH of the different administered nicotine forms is readily calculated by the skilled man in the art. The extent and duration of the increase in pH is dependent on type and amount of the buffering agent(s) used as well as where is further described within the paragraphs below.

### The active ingredient

According to the invention, the present oral formulation comprises nicotine in any form (for example free base, salt or complex).

With nicotine it is intended to include nicotine, 3-(1-methyl-2-pyrrolidinyl)-pyridine, with its base form, including synthetic nicotine as well as nicotine extracts from tobacco plants, or parts thereof, such as the genus Nicotiana alone or in combination; or pharmaceutically acceptable salts.

The nicotine compound should be in a saliva soluble form or facilitate a rapid release of the nicotine into the saliva in the oral cavity and, further, the subsequent uptake of the nicotine from the saliva in the oral cavity into the systemic circulation of the subject. When the nicotine prevails in the form of nicotine resinate complex, NRC, its release of nicotine is modified in the presence of a buffer.

In preferred embodiments, the nicotine in any form is selected from the group consisting of the free base form of nicotine, a nicotine salt, a nicotine derivative, such as a nicotine cation exchanger, a nicotine inclusion complex or nicotine in any non-covalent binding, nicotine bound to zeolites, nicotine bound to cellulose or starch micro spheres, and mixtures thereof.

Numerous nicotine salts are known, and may be used, e g the salts presented in Table 2, preferably monotartrate, hydrogen tartrate (also called bitartrate or bitartrate dihydrate), citrate, malate, and/or hydrochloride.

**Table 2 Possible acids used for nicotine salt formation**

| **Acid** | **Molar ratio* of acid:nicotine** |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

| | |
|---|---|
| * recommended at the time of production | |

The inclusion complex may be a nicotine-cyclodextrin (1-1) compound, such as nicotine-β-cyclodextrin.

Suitable cation exchangers are given in below Table 3 and are further disclosed in U.S. Patent Number 3,845,217. Preferred are nicotine cation exchangers of polyacrylates, such as the Amberlite collection from Rohm & Haas.

**Table 3 Representative cation exchangers**

| **Name** | **Type of crosslinked polymer** | **Manufacturer** |
|---|---|---|
| Amberlite IRC 50 | Divinylbenzene-methacrylic acid | Rohm & Haas |
| Amberlite IRP 64 | Divinylbenzene-methacrylic acid | Rohm & Haas |
| Amberlite IRP 64M | Divinylbenzene-methacrylic acid | Rohm & Haas |
| BIO-REX 70 | Divinylbenzene-acrylic acid | BIO-RAD Lab. |
| Amberlite IR 118 | Styrene-divinylbenzene | Rohm & Haas |
| Amberlite IRP 69 | Styrene-divinylbenzene | Rohm & Haas |
| Amberlite IRP 69M | Styrene-divinylbenzene | Rohm & Haas |
| BIO-REX 40 | Phenolic | BIO-RAD Lab. |
| Amberlite IR 120 | Styrene-divinylbenzene | Rohm & Haas |
| Dowex 50 | Styrene-divinylbenzene | Dow Chemical |
| Dowex 50W | Styrene-divinylbenzene | Dow Chemical |
| Duolite C 25 | Styrene-divinylbenzene | Chemical Process Co |
| Lewatit S 100 | Styrene-divinylbenzene | Farbenfabriken Bayer |
| Ionac C 240 | Styrene-divinylbenzene | Ionac Chem. |
| Wofatit KP S 200 | Styrene-divinylbenzene | I.G. Farben Wolfen |
| Amberlyst 15 | Styrene-divinylbenzene | Rohm & Haas |
| Duolite C-3 | Phenolic | Chemical Process |
| Duolite C-10 | Phenolic | Chemical Process |
| Lewatit KS | Phenolic | Farbenfabriken Bayer. |
| Zerolit 215 | Phenolic | The Permutit Co. |
| Duolite ES-62 | Styrene-divinylbenzene | Chemical Process |
| BIO-REX 63 | Styrene-divinylbenzene | BIO-RAD Lab. |
| Duolite ES-63 | Styrene-divinylbenzene | Chemical Process |
| Duolite ES-65 | Phenolic | Chemical Process |
| Ohelex 100 | Styrene-divinylbenzene | BIO-RAD Lab. |
| Dow Chelating Resin A-1 | Styrene-divinylbenzene | Dow Chemical Company |
| CM Sephadex C-25 | Dextran | Pharmacia Fine Chemicals |
| SE Sephadex C-25 | Dextran | Pharmacia Fine Chemicals |

One or more additives may be added to the present oral formulation. Additives are further described in the below paragraph *Other additives to the oral formulation..*

### Amount and distribution of the nicotine in the oral formulation

The nicotine in any form according to the invention is formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of reduction of said urge and providing a sense of smoking satisfaction without smoking. The amount of the nicotine should be sufficient to provide such an effect in a subject. This amount may, of course, vary from person to person.

According to the invention, embodiments of the oral formulation comprise embodiments wherein nicotine in any form is present in an amount of 0.05 - 8 mg calculated as the free base form of nicotine per unit dose of the oral formulation. This may in different embodiments include 0.1, 0.5, 1, 2, 3, 4, 5, 6 or 8 mg calculated as the free base form of nicotine per unit dose.

Still preferred embodiments may contain embodiments where the nicotine in any form is present in an amount of 0.5 - 6 mg calculated as the free base form of nicotine per unit does of the oral formulation.

Even more preferred embodiments contain the nicotine in any form in an amount of 0.5 - 5 mg calculated as the free base form of nicotine per unit dose of the oral formulation.

The nicotine in any form may be distributed in the oral formulations in different embodiments. Different distributions of the nicotine throughout the oral formulations will imply administration of the nicotine to the subject in different ways. This may, then, provide several possibilities to adjust the composition of the oral formulation according to different needs of different subjects depending on the urge to smoke or use tobacco of the subject. In the below Examples are disclosed different such embodiments.

### Other additives to the oral formulation

A strong base, e g sodium hydroxide, may be added to the formulation in order to give it a weak basic property.

Other additives may be added optionally to the oral formulation. Optional additives comprise at least one or more additives selected from the group consisting of solvents, such as ethanol and water; co-solvents, such as propylene glycol; stabilisers, such as preservatives, e g antioxidants; softeners, such as sorbitol and glycerine; thickening agents, such as colloidal silicon dioxide; binding agents, such as xanthan gum; filling agents, such as mannitol, isomalt, cocoa powder and Crospovidone; solubilizers, such as Polysorbat 80 and Atmos 300; rubbers, lipid barriers, such as sucrose fatty acid esters and hydrogenated vegetable oils; film forming agents, such as porcine gelatine, Pullulan, carrageenan, pectin, locust bean gum and xanthan gum; emulsifiers, such as pectin, soy lecithin, glycerol monostearate, castor oil and poloxamer; glidants, such as colloidal silicon dioxide; lubricants, such as magnesium stearate; coating agents, such as castor oil and sorbitol; melting vehicles, such as vegetable oils; sweeteners, flavours, aromatics, cooling agents, enhancers, colouring agents, vitamins, minerals, fluorine, breath fresheners, tooth whitening agents and mixtures thereof. According to the invention, at least one of such additives is optionally added to the product.

Enhancers may be added essentially to increase the transmucousal uptake of nicotine from the oral cavity.

Sweeteners are added essentially to improve the taste. Sweeteners comprise one or more synthetic or natural sugars, i e any form of carbohydrates suitable for use as sweetener, as well as so called artificial sweeteners such as saccharin, sodium saccharin, aspartame, e g NutraSweet^{®}, acesulfame or Acesulfame K, potassium acesulfame, thaumatin, glycyrrhizin, sucralose, dihydrochalcone, alitame, miraculin, monellin, stevside and neotame.

Suitable sweeteners may be selected from the group consisting of sugar alcohols, such as sorbitol, xylitol, single sugars including sugars extracted from sugar cane and sugar beet (sucrose), dextrose (also called glucose), fructose (also called leavulose), and lactose (also called milk sugar); sorbitol, mannitol, glycerol, xylitol, erythritol, maltitol syrup (or hydrogenated starch hydrolyzate), isomalt, lactitol; and mixtures of sugars including glucose syrup, e g starch hydrolysates, containing a mixture of dextrose, maltose and a range of complex sugars, invert sugar syrup, e g sucrose inverted by invertase (also called sucrase or sacchrase) containing a mixture of dextrose and fructose, high sugar content syrups such as treacle and honey containing a mixture of particular leavulose, dextrose, maltose, lactitole, sucrose, resins, dextrin and higher sugars; and malt or malt extracts.

The flavour and aroma additives may comprise one or more synthetic or natural taste-masking, flavouring or aromatizing agents. Flavour and aroma agents may be selected from essential oils including distillations, solvent extractions, or cold expressions of chopped flowers, leaves, peel or pulped whole fruit comprising mixtures of alcohols, esters, aldehydes and lactones; essences including either diluted solutions of essential oils, or mixtures of synthetic chemicals blended to match the natural flavour of the fruit, e g strawberry, raspberry and black currant; artificial and natural flavours of brews and liquors, e g cognac, whisky, rum, gin, sherry, port, and wine; coffee, tea, cocoa, and mint; fruit juices including expelled juice from washed, scrubbed fruits such as lemon, orange, and lime; spear mint, pepper mint, wintergreen, cinnamon, cacoe/cocoa, vanilla, liquorice, menthol, eucalyptus, aniseeds, nuts (e g peanuts, coconuts, hazelnuts, chestnuts, walnuts, colanuts), almonds, raisins; and powder, flour, or vegetable material parts and ginger.

Colouring additives may be selected from dyes being approved as a food additive.

Stabilizing additives may be selected from the group consisting of antioxidants including vitamin E, i.e. tocopherole, ascorbic acid, sodium pyrosulfite, butylhydroxytoluene, butylated hydroxyanisole, edetic acid and edetate salts; and preservatives including citric acid, tartaric acid, lactic acid, malic acid, acetic acid, benzoic acid, and sorbic acid. Preferred embodiments comprise an antioxidant as the stabiliser, and even more preferably the antioxidant vitamin E and/or butylated hydroxytoluene (BHT).

### Method for delivering nicotine in any form to a subject

The invention may be used to deliver nicotine to the subject (person) in a variety of ways. Described herein is a method for delivering nicotine in any form to a subject comprises the steps of:
a) administering to a subject an oral formulation containing nicotine in any form according to the invention into the oral cavity of the subject, and
b) if needed allowing the nicotine in any form in the oral formulation to be released in the saliva in the oral cavity and absorbed into the blood plasma of the subject. The method for delivering nicotine in any form may further comprise the steps of:
c) administering the nicotine in any form in a sustained way over a period of time to the subject. Such a time period may be at least 5, 10, 20, 30 or 40 minutes.

### Method for obtaining reduction of the urge to smoke or use of tobacco

Another feature of the invention is the ability to use the invention to reduce the urge to smoke. A use for obtaining reduction of the urge to smoke or use nicotine-containing tobacco products and/or for providing a sense of smoking satisfaction without smoking according to the invention comprises the steps of:
a) replacing at least partly the nicotine-containing tobacco product with an oral nicotine-containing formulation devoid of tobacco,
b) administering to a subject an oral formulation containing nicotine in any form into the oral cavity of the subject, and
c) if needed allowing the nicotine in any form in the oral formulation to be released in the saliva in the oral cavity and absorbed by the subject.

In another embodiment, the use according to the invention further comprises the steps of administering the nicotine in any form in a sustained way over a period of time to the subject. The period of time may be at least 5, 10, 20, 30 or 40 minutes.

Further embodiments of the use for delivering nicotine to a subject may comprise the steps of combining administration of the oral formulation with at least one other method for obtaining reduction of the urge to smoke or use of tobacco.

Tobacco containing material may be material used for e.g. smoking, snuffing or chewing and may comprise a cigarette, a cigar, pipe tobacco, snuff, snus and chewing tobacco.

### Sustained reduction of the urge to smoke or use of tobacco

The invention may also be used to reduce the urge to smoke or use tobacco. Still, to continue the feeling or sense of satisfaction of the subject, and to avoid that the craving returns, a sustained craving relief may be obtained after the initial craving relief. A sustained craving relief is obtained by using the oral formulation in such a way as to allow a sustained uptake of the nicotine. The sustained craving relief and/or feeling or sense of satisfaction of the subject will continue as long as the subject maintains the blood plasma levels of nicotine at a level high enough to reach this sense of feeling.

The subject may achieve this by using the oral formulation over a period of time, such as 5, 10, 20, 30 or 40 minutes or longer, e.g. a slow release of the nicotine caused by a controlled release, e.g. by individual use.

### Cessation of the urge to smoke or use of tobacco

For some of the users, it may be a goal to terminate the usage of nicotine completely, due to several reasons e.g. health, economical, social or behavioural. This cessation of smoking or the urge to use tobacco may be achieved by further decreasing the amount of nicotine in any form gradually over time. In a specific embodiment of the invention, the method described above for obtaining craving relief may further comprise the steps of decreasing the amount of nicotine in the oral formulation described above gradually over time, so as to achieve a complete relief of tobacco craving. This method results in a weaning process gradually over time.

Different types of smokers reach the sense of reduced craving at different plasma levels of nicotine. This may, of course, affect the individual types of administration programs of an oral formulation according to the invention. Different types of smokers include e g peak seekers or smokers that crave for a plasma level of nicotine constantly being above the level for withdrawal symptoms.

One strategy may be to lower the frequency of the administered oral formulation. Other embodiments include varying the dose of the nicotine in said oral formulations as well as the combination of these two. Also, the strategy may include an oral formulation with substantially no nicotine in any form. Such an oral formulation may be administered at the end of the treatment period, when the craving is low or substantially absent.

### Systems for delivering nicotine and for obtaining craving relief

According to the invention there is a system according to the claims for delivering nicotine in any form to a subject particularly for obtaining craving relief. Such a system comprises an oral formulation according to the invention and at least one other means for obtaining reduction of the urge to smoke.

Another system according to the invention may also be a system for obtaining reduction of the urge to smoke or use of tobacco and/or for providing a sense of smoking satisfaction without smoking. Such a system comprises an oral formulation according to the invention and at least one other method or means for obtaining reduction of the urge to smoke or use tobacco. Other methods and means may also be a concomitant or concurrent method selected from the group consisting of administration through mouth sprays, nasal sprays, transdermal patches, inhaling devices, lozenges, tablets and parenteral methods, subcutaneous methods, and transmucousal methods; or use of tobacco.

In a specific embodiment, the at least one other method comprises administration of nicotine.

### Use of the oral formulation

The use of the oral formulation according to the invention may include obtaining a fast and/or sustained and/or complete reduction of the urge to smoke and use tobacco or for providing a sense of smoking without smoking as described above.

The dose of the nicotine is chosen to give the subject an individual sensory perception and satisfaction with an effect of the nicotine in any form. The use of an oral formulation may also be a sole use according to the invention or a combination with other means or methods known in the field of drug abuse. Specifically, the present invention may be used in combination with other means as described above in the methods in the paragraphs above.

The use may give a quick reduction of the urge to smoke or use tobacco. Other embodiments include providing a slow reduction of the urge to smoke or use tobacco.

### Use for therapy and treatment

The oral formulation according to the invention may be used in therapy and treatment. Said therapy may be a treatment of a disease selected from the group consisting of tobacco or nicotine dependence, Alzheimer's disease, Crohn's disease, Parkinson's disease, Tourette's syndrome, ulcerous colitis and post-smoking-cessation weight control.

Nicotine may also be used for an oral formulation according to the invention for the treatment of said diseases.

Further, nicotine may be used in the production of a nicotine-containing oral formulation according to the invention for the treatment of said diseases.

### Production of the oral formulation

The oral formulations according to the present invention are basically produced according to methods known in the art. Exemplary, but not limiting, production methods are provided below under Examples.

In the below Examples is described mixing, rolling and scoring of chewing gums as well as compression of chewing gums. The below Examples also provide information on manufacturing of other embodiments of the present invention.

Conveniently, the compositions of additives according to the invention, e g the buffer system, are made simultaneously, according to known procedures in the art for formulating e g the buffers. Depending on the physical properties of the buffer system incorporated, it may be convenient to add the buffer system/s either with the liquid part or with the solid part of the composition. In the case of buffering systems available as fine powders, it may, of course, be most convenient to add those powders with the solid, powdered part of other additives. The final product may then be analysed and wrapped.

### Analysis of nicotine

The analysis of nicotine uptake and effect according to the invention may be done according to standard procedures known in the art, e g using bioanalysis for the determination of nicotine or its metabolites in the plasma of a subject.

### Examples

The below examples on embodiments of the present invention are illustrative and non-limiting. The skilled person may on the basis of the following examples envisage also other embodiments of the present invention. Batch sizes for the manufacture of the below formulations may be modified according to the actual need and to the actual production facilities. If not stated otherwise procedures and equipment known in the art are used in the below manufacturing.

In the below Examples the amino acids used are L-Arginine, Glycine and N-glycylglycine. The skilled person may though exchange L-Arginine, Glycine and N-glycylglycine for one or more other amino acids, such as amino acids selected from above Table 1, thereby adapting the amount(s) of amino acid according to state of the art methods. Further, the skilled person may readily calculate whether a pH-adjusting compound need be added. Accordingly in some of the below Examples a pH-adjusting compound has been added in addition to the buffer.

### Example 1 Tablets

### 275 mg tablet with 2 mg nicotine

| **Ingredients** | **Amount in composition (mg)** |
|---|---|
| Nicotine bitartrate dehydrate | 6,1 |
| L-Arginine | 17.9 |
| Mannitol | 210.9 |
| Xanthan gum | 11,0 |
| Crospovidone | 11,0 |
| Flavoring agents | 9,9 |
| Aspartame | 1,6 |
| Acesulfame K | 1,1 |
| Magnesium stearate | 5,5 |

### Manufacturing process:

The above ingredients are blended. The blend is then compressed into tablets by means of direct compression according to methods known in the art.

### Example 2 Melt tablets

This is a tablet intended for melting in the mouth whereupon the melted material adheres to the oral mucosa where the nicotine is deposited for entering into the tissue.

### 400 mg melt tablet with 2 mg nicotine

| **Ingredients** | **Relative amount in composition (%w/w)** |
|---|---|
| Nicotine bitartrate dehydrate | 1.5 |
| Cocoa powder | 35.0 |
| Vegetable oil | 41.6 |
| L-Arginine | 4.5 |
| Mannitol | 10.4 |
| Titanium dioxide | 2.7 |
| Soy lecithin | 1.0 |
| Aspartame | 0.4 |
| Acesulfame K | 0.2 |
| Flavoring agents | 2.7 |

### Manufacturing process:

The manufacturing as such takes place at room temperature. A part of the fatty component, i e the vegetable oil, is melted. The solid components, i e the nicotine salt, the cocoa powder, the buffering agent, the mannitol, the titanium oxide, the sweeteners and the flavoring agents are added and mixed. A reduction of particle size of the solid components is performed by milling the mixture in a roll-refiner. If the solid components have already got the required particle size, e g by milling before the mixing with the fatty component, roll refining is dispensed with. After possible treatment in the roll-refiner the mixture is mixed with the rest of the melted vegetable oil or remelted (if solidified) and mixed with the rest of the melted vegetable oil. A mixing of the melt is performed in a suitable mixer. The liquid component, i e the soy lecithin, is added.

Tablets are subsequently made using suitable techniques, such as molding, extrusion or congealing, including pastillation, when necessary after suitable preconditioning. Also other suitable manufacturing methods known in the art may be used.

### Example 3A Mouth spray

**Nicotine mouth spray with 14.3 mg nicotine/ml and pH 9.0**

| **Ingredients** | **mg/ml** |
|---|---|
| Nicotine free base | 14.3 |
| Ethanol | 100.0 |
| Propylene glycol | 150.0 |
| Glycerine | 25.0 |
| L-Arginine | 58.3 |
| Sodium Hydrogen Carbonate | 14.3 |
| Poloxamer | 40.0 |
| Levomenthol | 10.0 |
| Flavoring agent | 4.0 |
| Cooler | 3.0 |
| Sweeteners | 3.0 |
| Hydrochloric acid | Ad pH 9.0 |
| Purified water | q.s. |

### Example 3B Mouth spray

### Nicotine mouth spray with 14.3 mg nicotine/ml and pH 9

### Batch size: 1000 ml

| Ingredients | mg/ml |
|---|---|
| Nicotine free base | 14.3 |
| Ethanol | 100.0 |
| Propylene glycol | 150.0 |
| Glycerine | 25.0 |
| Glycine comparative or L-Arginine | 25.0 or 58.3 |
| Sodium Hydrogen Carbonate | 14.3 |
| Poloxamer | 40.0 |
| Levomenthol | 10.0 |
| Flavouring agent | 4.0 |
| Cooler | 3.0 |
| Sweeteners | 3.0 |
| Hydrochloric acid | Ad pH 9.0 |
| Purified water | q.s. |

In Examples 3A and 3B, and in some of the following Examples, the pH-adjusting capacity of the amino acid buffer is increased by the addition of an adequate amount of pH-adjusting compound.

### Manufacturing process:

### Mixture I

1. Purified water is charged into a vessel.
2. Poloxamer is added which shall be solved during mixing before entering next step.
3. Sweeteners, Glycine (or L-Arginine) and Sodium Hydrogen Carbonate are added.
4. Continue with the stirring until all components are dissolved.
   Mixture II
5. Ethanol is charged into a vessel.
6. Levomenthol is added and the stirring is ongoing until dissolved.
7. Propylene glycol, Flavoring agent, Glycerine and Cooler are added. The agitation is continued until the solution is homogeneous.
   Final mixing
8. Pour Mixture II into Mixture I and stir until a homogeneous solution is reached.
9. Nicotine is added to the solution while gently stirring.
10. Measure pH of the solution. Adjust pH to 9 by adding Hydrochloric acid.
11.Purified water is added q.s. to batch quantity. The solution is mixed until a clear solution is obtained.

### Example 4 Capsules

### Nicotine soft capsules 2 mg

| **Ingredients** | **% (w/w)** |
|---|---|
| *Ingredients of Core:* | |
| Nicotine free base | 2.2 % |
| Mediuin chain triglycerides | 86.6% |
| Flavors and sweeteners | 7.8 % |
| L-Arginine | 2.9 % |
| Colloidal silicon dioxide | 0.5 % |
| | |
| *Ingredients of Inner Shell:* | |
| Sucrose fatty acid ester | 60.0% |
| Hydrogenated vegetable oil | 40.0% |
| | |
| *Ingredients of Outer Shell:* | |
| Porcine gelatin | 80.0% |
| Sorbitol | 18.0% |
| Glycerin | 2.0% |
| | |
| *Weight Ratio:* | |
| *Core*/*Inner shell*/*Outer shell* | 64/30/6 |
| *Total Capsule weight:* | 142 mg |

### Use:

Seamless soft gel capsules are soft gelatin capsules that are distinguished by their spherical shape and thin, seamless gelatin shell. The thin shell makes the capsules suitable for use in orally dissolving products compared to conventionally produced soft gelatin capsules that are intended to be chewed or swallowed.

### Manufacturing process:

Seamless soft gel capsules are manufactured by formation of droplets consisting of two or more concentric layers. The droplets are formed by feeding different liquids through concentric nozzles. The outermost nozzle feeds a hydrophilic solution consisting of gelatin and additives e g plasticizers. The one or more inner nozzles feed a lipophilic liquid (e g oils, triglycerids) wherein one or more active substances may be dispersed. The lipophilic centre and hydrophilic perimeter of the formed droplets ensure a good phase separation between shell and core contents. The formed capsules are then subjected to sequential processing steps such as cooling, drying, washing and selection of size and shape.

### Example 5A Hard-boiled candies

### Nicotine hard-boiled candy with 2 mg nicotine

| **Ingredients** | **% (w/w)** |
|---|---|
| Purified water | - |
| Isomalt | 78.0 |
| Maltitol 75 % solution | 19.5 |
| Nicotine bitartrate dihydrate | 0.2 |
| L-Arginine | 1,6 |
| Flavor | 0.7 |
| | |
| Total | 100.0 |

Piece weight 3.5 g
Nicotine /piece 2 mg

### Manufacturing process:

1. To a stainless steel beaker add purified water, isomalt and maltitol solution. Mix and heat during continuous mixing.
2. Discontinue heating and cool to 135-140 °C. Add nicotine bitartrate dihydrate and mix until fully dispersed. Add L-Arginine and mix at 120 °C until dispersed.
3. Add flavor, mix until uniform.
4. Pour into molds, let cool.

### Example 5B Hard-boiled candies

### Nicotine hard-boiled candy with 2 mg nicotine

| **Ingredients** | **% (w/w)** |
|---|---|
| Purified water | |
| Isomalt | 78.0 |
| Maltitol 75 % solution | 15.2 |
| Nicotine bitartrate dihydrate | 0.6 |
| N-glycylglycine | 3.2 |
| Sodium carbonate | 1.0 |
| Flavour | 2.0 |
| Total | 100.0 |

Piece weight 1.0 g
Nicotine /piece 2 mg

### Manufacturing process:

1. To a stainless steel beaker add purified water, isomalt and maltitol solution. Mix and heat during continuous mixing.
2. Discontinue heating and cool to 135-140 °C. Add nicotine bitartrate dihydrate and mix until fully dispersed. Add N-glycylglycine, sodium carbonate and mix at 120 °C until dispersed.
3. Add flavour, mix until uniform.
4. Pour into molds, let cool.

### Example 6 Oral films

### Nicotine bi-layer film with 2 mg nicotine

| **Ingredient** | **mg/film (Active part)** | **mg/film (Buffer part)** |
|---|---|---|
| Nicotine free base | 2.00 | - |
| Pullulan | 30.0 | 29.9 |
| Xanthan gum | 0.04 | 0.04 |
| Locust bean gum | 0.08 | 0.08 |
| Carrageenan | 0.38 | 0.38 |
| Pectin | 0.27 | 0.27 |
| Polysorbat 80 | 0.46 | 0.46 |
| Atmos 300 | 0.46 | 0.46 |
| Sucralose | 2.10 | 2.10 |
| Sorbitol | 2.90 | 2.90 |
| Menthol | 1.95 | 1.95 |
| Flavor | 12.7 | 12.7 |
| Colouring agent | 0.02 | 0.02 |
| L-Arginine | - | 4.09 |
| Sodium carbonate anhydrous | - | 1.23 |
| Tartaric acid | 3.7 | - |
| Purified water | About 4.20 | About 4.20 |
| Sub-Total | 60 | 60 |
| Total | 120 | |

### Manufacturing process (Part 1)

1. Mix together Pullulan, xanthan gum, locust bean gum, carrageenan and pectin.
2. Add heated water to the mixture
3. Add sucralose and sorbitol, mix to dissolve. Cool to room temperature.
4. Pre-mix Polysorbate 80, Atmos 300, colouring agent, menthol and flavor, and add to the blend.
5. Add tartaric acid and then nicotine free base and mix.
6. Cast Pullulan solution onto substrate of desired thickness and dry with hot air.

### Manufacturing process (Part 2):

1. Mix together Pullulan, xanthan gum, locust bean gum, carrageenan, pectin, L-Arginine and sodium carbonate.
2. Add heated water to the mixture.
3. Add sucralose and sorbitol, mix to dissolve. Cool to room temperature.
4. Pre-mix Polysorbate 80, Atmos 300, colouring agent, menthol and flavor, and add to the blend
5. Cast Pullulan solution onto substrate of desired thickness and dry with hot air. Manufacturing (Part 3)
   1. Cast film (Active) and cast film (Buffer) are laid upon each other and slightly pressed together.
   2. Cut into desired size. Size of e g 24 mm x 33 mm is appropriate.

If necessary a barrier layer may be placed between the cast active-containing film and the cast buffer-containing film in order to avoid chemical reactions between these two films.

Also multi-layer oral films are envisageable.

### Example 7 Gummies

### Nicotine gummy with 1 mg nicotine

| **Ingredients** | **g per piece** |
|---|---|
| Isomalt | 3.7 |
| Sweetener | 1.0 |
| Water | 0.1 |
| Pectin | 0.1 |
| L-Arginine | 0.036 |
| Flavor | 0.1 |
| Nicotine bitartrate dihydrate | 0.0032 |
| Total | 5 g |

### Manufacturing process:

1. Heat the isomalt to melting point and add sweetener and let the mixture cool.
2. To the cooled mixture, add pectin solution, L-Arginine and flavor.
3. Add nicotine bitartrate dihydrate, mix thoroughly.
4. Cast using starch moulds in desired shape and size using methods known in the art.

### Example 8 Chewy candies

### Nicotine chewy candy with 1 mg nicotine

| **Ingredients** | **g per piece** |
|---|---|
| Isomalt | 3.4 |
| Sweetener | 1.0 |
| Water | 0.1 |
| Vegetable oil | 0.3 |
| Glycerol monostearate | 0.1 |
| L-Arginine | 0.036 |
| Flavor | 0.1 |
| Nicotine bitartrate dihydrate | 0.0032 |
| Total | 5 g |

### Manufacturing process

1. Heat the isomalt to melting point and add sweetener and let the mixture cool.
2. To the cooled mixture, add vegetable oil, L-Arginine and flavor. Mix well.
3. Add nicotine bitartrate dihydrate. Mix well. Cast in molds or extrude and cut to desired size using methods known in the art.

### Example 9A Compressed chewing gums

### Example 9A

### Nicotine-containing compressed chewing gum with 2 mg nicotine

| **Ingredients** | **Amount in composition (mg)** |
|---|---|
| Nicotine resin complex (NRC) 20% | 10 |
| Chewing gum base | 556 |
| L-Arginine | 36 |
| Sodium carbonate | q.s. |
| Castor oil | 60 |
| Sorbitol | 140 |
| Flavoring agents | 118 |
| Sweeteners | 5 |
| Colloidal silicon dioxide | 22.5 |
| Magnesium stearate | 20 |
| Talc | 22.5 |
| Sodium hydrogen carbonate | q.s. |

### Manufacturing process:

1) The nicotine resin complex is blended with the hydrophilic aqueous soluble element, i e sorbitol.
2) The hydrophobic element, which is practically insoluble in water, i e castor oil, is heated to a suitable temperature until a solution is obtained.
3) The blend obtained in 1) is added to the solution 2) under vigorous stirring.
4) The above blend in 3) is cooled to below room temperature and blended with gum base and other additives.
5) The above blend in 4) is, if necessary, sieved to remove aggregates and compressed into gums by means of direct compression.

### Example 9B

### Nicotine compressed chewing gum with 2 mg nicotine

| **Ingredients** | **Amount in composition (mg)** |
|---|---|
| Nicotine resin complex 20% | 10 |
| Chewing gum base | 300 |
| L-Arginine | 36 |
| Sodium carbonate | q.s. |
| Isomalt | 95 |
| Sorbitol | 491 |
| Flavouring agents | 30 |
| Sweeteners | 3 |
| Colloidal silicon dioxide | 5 |
| Magnesium stearate | 20 |
| Sodium hydrogen carbonate | q.s. |

### Manufacturing process description:

1) Mixing: A gum base powder mixture comprising gum base, sweeteners and glidant is blended with active, flavoring agent, glidant, artificial sweeteners, buffering agents and lubricant.
2) Tableting: The above blend is sieved to remove aggregates if necessary and compressed into gums by means of direct compression.

### Example 10 Chewing gums made by mixing, rolling and scoring

| **Ingredients** | **2 mg Unit formula (mg)** | **4 mg Unit formula (mg)** |
|---|---|---|
| Nicotine resin complex 20% | 10 | 20 |
| Chewing gum base | 657 | 650 |
| Xylitol | 250 | 230 |
| L-Arginine | 43 | 65 |
| Flavouring agents | 32 | 22 |
| Levomenthol | 2 | 2 |
| Magnesium oxide | 1 | 1 |

### Manufacturing process:

Mixing, rolling and scoring is done by a conventional procedure. Double sigma blade mixers are used for mixing the gum base with the other components of the formulation. The gum base is softened in the mixer. By heat (from the heating jacket) and mixing, the gum base becomes plastic. So, the softened base is mixed with the liquid components and the solid materials as a powder mixture. The warm mass is discharged from the mixer in form of loaves stacked on trays on a truck and stored in a conditioned area until the next step starts. This is to cool the gum.

After this, the rolling and scoring takes place. The gum is extruded into a thick sheet, which is rolled by multiple sets of calendar rolls to the correct thickness. The scoring rolls, usually two sets, cut the sheet into correctly sized pieces.

The sheets are then transferred to a conditioned area on trays, where the sheets are cooled to make them brittle enough to be broken. The conditioned gum sheets are then passed through a breaker, which is a rotating drum that parts the sheets into separate pieces of gum along the scores.

At a sorting stage deformed gums are sorted out. The so accepted gums are further checked by being passed by a metal detector.

## Claims

1. A buffered pharmaceutical oral formulation comprising nicotine, **characterized in that** it is buffered with at least one amino acid and/or a salt thereof, and **in that** it comprises a pH-adjusting compound when the pH-adjusting capacity of said at least one amino acid and/or a salt thereof is insufficient;
wherein the at least one amino acid and/or a salt thereof comprises an endogenous amino acid which is selected from the group consisting of Arginine, Aspargine, Glutamic acid, Glutamine, Histidine, Isoleucine, Leucine, Lysine, Methionine, Ornithine, Phenylalanine, Serine, Threonine and Valine; and
wherein the oral formulation is devoid of tobacco.

2. The oral formulation according to claim 1, **characterized in that** the at least one endogenous amino acid is/are chosen among Aspargine, Glutamic acid, Glutamine, Histidine, Isoleucine, Leucine, Methionine, Phenylalanine, Serine, Threonine and Valine.

3. The oral formulation according to any preceding claim, being buffered in such a way that upon oral administration of the oral formulation to a subject the pH of the saliva of the subject is increased by 0.2 - 4 pH units, or by 0.5 - 2 pH units.

4. The oral formulation according to any preceding claim being buffered with an amino acid together with one or more buffers and/or one or more pH adjusting compounds selected from the group consisting of a carbonate including bicarbonate or sesquicarbonate, glycinate, phosphate, glycerophosphate or citrate of an alkali metal, such as potassium or sodium, or ammonium, including trisodium phosphate, disodium hydrogen phosphate; tripotassium phosphate, dipotassium hydrogen phosphate, and calcium hydroxide, sodium glycinate, trometamol; and mixtures thereof.

5. The oral formulation according to any of claims 1 - 4, wherein the nicotine in any form is selected from the group consisting of a nicotine salt, the free base form of nicotine, a nicotine derivative, such as a nicotine cation exchanger, a nicotine inclusion complex or nicotine in any non-covalent binding; nicotine bound to zeolites; nicotine bound to cellulose or starch micro-spheres; and mixtures thereof.

6. The oral formulation according to claim 5, wherein the nicotine inclusion complex is a cyclodextrin complex, such as ß-cyclodextrin.

7. The oral formulation according to claim 5, wherein the nicotine cation exchanger is a polyacrylate cation exchanger.

8. The oral formulation according to claim 5, wherein the nicotine salt is a monotartrate, hydrogen tartrate, citrate, malate and/or hydrochloride salt.

9. The oral formulation according to any of claims 1 - 10, wherein the nicotine in any form is present in an amount of 0.05 - 8 mg or 0.1 - 6 mg or 2 - 5 mg calculated as the free base form of nicotine per unit dose.

10. The oral formulation according to any of claims 1 - 9, wherein optionally at least one or more additive is selected from the group consisting of solvents, co-solvents, stabilisers, preservatives, antioxidants, softeners, thickening agents, binding agents, filling agents, solubilizers, rubbers, lipid barriers, film forming agents, emulsifiers, glidants, lubricants, coating agents, melting vehicles, sweeteners, flavors, aromatics, cooling agents, enhancers, colouring agents, vitamins, minerals, fluorine, breath fresheners, tooth whitening agents and mixtures thereof.

11. The oral formulation according to any of claims 1 - 10 being in the form of a mouth spray, a capsule, a chewing gum, a chewable tablet, a tablet, a melt tablet, a lozenge, a hard boiled candy, a chewy candy, a gummy, or an oral film.

12. The oral formulation according to any of claims 1 - 11 being non-coated.

13. An oral formulation according to any one of claims 1 to 4 wherein the oral formulation is a nicotine-containing tablet comprising nicotine bitartrate dihydrate, an amino acid, mannitol or filling agent, xanthan gum or another binder, crospovidone or another disintegrant, one or more flavors, and one or more artificial sweeteners.

14. An oral formulation according to any one of claims 1 to 4 wherein the oral formulation is a nicotine-containing melt tablet comprising nicotine bitartrate dihydrate, cocoa powder or another filler/texturizer/taste masker, vegetable oil or another melting vehicle, an amino acid, mannitol or another diluent, soy lecithin or another emulsifier, coloring agent, artificial sweeteners and flavoring agents.

15. An oral formulation according to any one of claims 1 to 4 wherein the oral formulation is a nicotine-containing mouth spray comprising nicotine free base, ethanol or another solvent, an amino acid, poloxamer or another solubilizer, tetracemindinatrium or another stabilizer, and artificial sweeteners.

16. An oral formulation according to any one of claims 1 to 4 wherein the oral formulation is a nicotine-containing soft capsule comprising in the core nicotine free base, medium chain triglycerides or another lipophilic vehicle, flavoring agents, an amino acid, and thickening agent, in the inner shell hydrophilic shell forming materials and in the outer shell forming material and softeners.

17. An oral formulation according to any one of claims 1 to 4 wherein the oral formulation is a nicotine-containing hard boiled candy comprising nicotine bitartrate dihydrate, isomalt, maltitol, an amino acid and flavoring agent.

18. An oral formulation according to any one of claims 1 to 4 wherein the oral formulation is a nicotine-containing oral film comprising nicotine bitartrate dihydrate, xanthan gum, locust bean gum, carrageenan, pectin, pullulan, an amino acid, polysorbate, artificial sweetening agent and flavoring agent.

19. An oral formulation according to any one of claims 1 to 4 wherein the oral formulation is a nicotine-containing gummy comprising nicotine bitartrate dihydrate, isomalt, pectin, an amino acid, artificial sweetening agent and flavoring agent.

20. An oral formulation according to any one of claims 1 to 4 wherein the oral formulation is a nicotine-containing chewy candy comprising nicotine bitartrate dihydrate, isomalt, vegetable oil, an amino acid, sweetening agent and flavoring agent.

21. An oral formulation according to any one of claims 1 to 4 wherein the oral formulation is a nicotine-containing chewing gum being manufactured through direct compressing comprising nicotine resin complex, gum base, an amino acid, and one or more of sweeteners, artificial sweeteners, glidants, flavoring agents and lubricants, and optionally hydrophobic agents.

22. An oral formulation according to any one of claims 1 to 4 wherein the oral formulation is a nicotine-containing chewing gum being manufactured through mixing, rolling and scoring comprising nicotine resin complex, gum base, sweetening agent, flavoring agents and an amino acid.

23. A system for delivering nicotine in any form to a subject, said system comprising an oral formulation according to any of claims 1 - 22 and at least one product comprising nicotine for obtaining reduction of the urge to smoke or use tobacco.

24. The system according to claim 23, wherein the at least one other product is selected from the group consisting of mouth sprays, nasal sprays, transdermal patches, inhaling devices, lozenges, tablets and tobacco.

25. An oral formulation according to claim 1 - 22 for use in obtaining a quick and/or sustained and/or complete reduction of the urge to smoke or use of tobacco and/or for providing a sense of smoking satisfaction without smoking.

26. An oral formulation according to any of claims 1 - 22 for use in delivering nicotine in any form to a subject.

27. A formulation according to any of claims 1 - 22 for use in therapy.

28. A formulation according to claim 27, wherein the therapy is treatment of a disease selected from the group consisting of tobacco or nicotine dependence, Alzheimer's disease, Crohn's disease, Parkinson's disease, Tourette's syndrome, ulcerous colitis and post-smoking-cessation weight control.

29. Use of nicotine for the production of an oral formulation according to any one of claims 1 - 22 for the treatment of a disease selected from the group consisting of tobacco or nicotine dependence, Alzheimer's disease, Crohn's disease, Parkinson's disease, Tourette's syndrome, ulcerous colitis and post-smoking-cessation weight control.

## Patentansprüche

1. Gepufferte pharmazeutische orale Formulierung, die Nicotin umfasst, **dadurch gekennzeichnet, dass** sie mit mindestens einer Aminosäure und/oder einem Salz davon gepuffert ist und dass sie eine den pH-Wert einstellende Verbindung umfasst, wenn die pH-Einstellungskapazität der mindestens einen Aminosäure und/oder eines Salzes davon unzureichend ist;
wobei die mindestens eine Aminosäure und/oder ein Salz davon eine endogene Aminosäure aus der Gruppe bestehend aus Arginin, Asparagin, Glutaminsäure, Glutamin, Histidin, Isoleucin, Leucin, Lysin, Methionin, Ornithin, Phenyl-alanin, Serin, Threonin und Valin umfasst und wobei die orale Formulierung tabakfrei ist.

2. Orale Formulierung nach Anspruch 1, **dadurch gekennzeichnet, dass** die mindestens eine endogene Aminosäure aus Asparagin, Glutaminsäure, Glutamin, Histidin, Isoleucin, Leucin, Methionin, Phenyl-alanin, Serin, Threonin und Valin ausgewählt ist.

3. Orale Formulierung nach einem der vorhergehenden Ansprüche, die so gepuffert ist, dass bei oraler Verabreichung der oralen Formulierungen an einen Empfänger der pH-Wert des Speichels des Empfängers um 0,2 - 4 pH-Einheiten oder um 0,5 - 2 pH-Einheiten erhöht wird.

4. Orale Formulierung nach einem der vorhergehenden Ansprüche, die mit einer Aminosäure zusammen mit einem oder mehreren Puffern und/oder einer oder mehreren den pH-Wert einstellenden Verbindungen aus der Gruppe bestehend aus einem Carbonat einschließlich Hydrogencarbonat oder Sesquicarbonat, Glycinat, Phosphat, Glycerophosphat oder Citrat von einem Alkalimetall, wie Kalium oder Natrium, oder Ammonium einschließlich Trinatriumphosphat, Dinatriumdihydrogenphosphat, Trikaliumphosphat, Dikaliumdihydrogenphosphat und Calciumhydroxid, Natriumglycinat Trometamol und Mischungen davon gepuffert ist.

5. Orale Formulierung nach einem der Ansprüche 1 - 4, wobei das Nicotin in beliebiger Form aus der Gruppe bestehend aus einem Nicotinsalz, der Form der freien Base von Nicotin, einem Nicotinderivat, wie einem Nicotin-Kationenaustauscher, einem Nicotin-Einschlusskomplex oder Nicotin in einer beliebigen nichtkovalenten Bindung, an Zeolithe gebundenem Nicotin, an Cellulose oder Stärke-Mikrokugeln gebundenem Nicotin und Mischungen davon ausgewählt ist.

6. Orale Formulierung nach Anspruch 5, wobei es sich bei dem Nicotin-Einschlusskomplex um einen Cyclo-dextrinkomplex, wie β-Cyclodextrin, handelt.

7. Orale Formulierung nach Anspruch 5, wobei es sich bei dem Nicotin-Kationenaustauscher um einen Polyacrylat-Kationenaustauscher handelt.

8. Orale Formulierung nach Anspruch 5, wobei es sich bei dem Nicotinsalz um ein Monotartrat-, Hydrogentartrat-, Citrat-, Malat- und/oder Hydrochlorid-Salz handelt.

9. Orale Formulierung nach einem der Ansprüche 1 - 10, wobei das Nicotin in beliebiger Form in einer Menge von 0, 05 - 8 mg oder 0, 1 - 6 mg oder 2 - 5 mg, berechnet als Form der freien Base von Nicotin, pro Dosiseinheit vorliegt.

10. Orale Formulierung nach einem der Ansprüche 1 - 9, wobei gegebenenfalls mindestens ein oder mehrere Additive aus der Gruppe bestehend aus Lösungsmitteln, Cosolventien, Stabilisatoren, Konservierungsmitteln, Antioxidantien, Weichmachern, Verdickungsmitteln, Bindemitteln, Füllstoffen, Löslichkeitsvermittlern, Kautschuken, Lipidbarrieren, Filmbildnern, Emulgatoren, Gleitmitteln, Schmiermitteln, Beschichtungsmitteln, schmelzenden Vehikeln, Süßungsmitteln, Geschmacksstoffen, Aromastoffen, kühlend wirkenden Mitteln, Verstärkern, Farbmitteln, Vitaminen, Mineralien, Fluor, Atemerfrischern, Zahnweißungsmitteln und Mischungen davon ausgewählt werden.

11. Orale Formulierung nach einem der Ansprüche 1 - 10 in Form eines Mundsprays, einer Kapsel, eines Kaugummis, einer Kautablette, einer Tablette, einer Schmelztablette, eine Lutschtablette, einer Hartkaramelle, einer Weichkaramelle, eines Gummis oder eines oralen Films.

12. Orale Formulierung nach einem der Ansprüche 1 - 11, die unbeschichtet ist.

13. Orale Formulierung nach einem der Ansprüche 1 bis 4, wobei es sich bei der oralen Formulierung um eine Nicotin enthaltende Tablette, die Nicotinbitartratdihydrat, eine Aminosäure, Mannitol oder Füllstoff, Xanthan oder ein anderes Bindemittel, Povidon oder ein anderes Zerfallsmittel, einen oder mehrere Geschmacksstoffe und ein oder mehrere künstliche Süßungsmittel umfasst, handelt.

14. Orale Formulierung nach einem der Ansprüche 1 bis 4, wobei es sich bei der oralen Formulierung um eine Nicotin enthaltende Schmelztablette, die Nicotinbitartratdihydrat, Kakaopulver oder ein/en anderen/s Füllstoff/Texturierungsmittel/Geschmacksmaskierungsmittel, Pflanzenöl oder ein anderes schmelzendes Vehikel, eine Aminosäure, Mannitol oder ein anderes Verdünnungsmittel, Sojalecithin oder einen anderen Emulgator, Farbmittel, künstliche Süßungsmittel und Geschmacksstoffe umfasst, handelt.

15. Orale Formulierung nach einem der Ansprüche 1 bis 4, wobei es sich bei der oralen Formulierung um ein Nicotin enthaltendes Mundspray, das Nicotin in Form der freien Base, Ethanol oder ein anderes Lösungsmittel, eine Aminosäure, Poloxamer oder einen anderen Löslichkeitsvermittler, Tetracemindinatrium oder einen anderen Stabilisator und künstliche Süßungsmittel umfasst, handelt.

16. Orale Formulierung nach einem der Ansprüche 1 bis 4, wobei es sich bei der oralen Formulierung um eine Nicotin enthaltende Weichkapsel, die im Kern Nicotin in der freien Base, mittelkettige Triglyceride oder ein anderes lipophiles Vehikel, Geschmacksstoffe, eine Aminosäure und Verdickungsmittel, in der inneren Hülle hydrophile Schalenbildungsmaterialien und in der äußeren Hülle Außenschalenbildungsmaterial und Weichmacher umfasst, handelt.

17. Orale Formulierung nach einem der Ansprüche 1 bis 4, wobei es sich bei der oralen Formulierung um eine Nicotin enthaltende Hartkaramelle, die Nicotinbitartratdihydrat, Isomalt, Maltitol, eine Aminosäure und Geschmacksstoff umfasst, handelt.

18. Orale Formulierung nach einem der Ansprüche 1 bis 4, wobei es sich bei der oralen Formulierung um einen Nicotin enthaltenden oralen Film, der Nicotinbitartratdihydrat, Xanthan, Johannisbrotkernmehl, Carrageenan, Pektin, Pullulan, eine Aminosäure, Polysorbat, künstliches Süßungsmittel und Geschmacksstoff umfasst, handelt.

19. Orale Formulierung nach einem der Ansprüche 1 bis 4, wobei es sich bei der oralen Formulierung um einen Nicotin enthaltenden Gummi, der Nicotinbitartratdihydrat, Isomalt, Pektin, eine Aminosäure, künstliches Süßungsmittel und Geschmacksstoff umfasst, handelt.

20. Orale Formulierung nach einem der Ansprüche 1 bis 4, wobei es sich bei der oralen Formulierung um eine Nicotin enthaltende Weichkaramelle, die Nicotinbitartratdihydrat, Isomalt, Pflanzenöl, eine Aminosäure, Süßungsmittel und Geschmacksstoff umfasst, handelt.

21. Orale Formulierung nach einem der Ansprüche 1 bis 4, wobei es sich bei der oralen Formulierung um einen Nicotin enthaltenden Kaugummi, der durch Direktverpressen hergestellt wird und Nicotin-Harz-Komplex, Kaugummigrundmasse, eine Aminosäure und ein oder mehrere Süßungsmittel, künstliche Süßungsmittel, Gleitmittel, Geschmacksstoffe und Schmiermittel und gegebenenfalls hydrophobe Mittel umfasst, handelt.

22. Orale Formulierung nach einem der Ansprüche 1 bis 4, wobei es sich bei der oralen Formulierung um einen Nicotin enthaltenden Kaugummi, der durch Mischen, Rollen und Erhitzen hergestellt wird und Nicotin-Harz-Komplex, Kaugummigrundmasse, Süßungsmittel, Geschmacksstoffe und eine Aminosäure umfasst, handelt.

23. System zur Zuführung von Nicotin in beliebiger Form an einen Empfänger, wobei das System eine orale Formulierung nach einem der Ansprüche 1 - 22 und mindestens ein Nicotin umfassendes Produkt umfasst, zur Erzielung einer Verringerung des Drangs zum Rauchen oder zur Verwendung von Tabak.

24. System nach Anspruch 23, wobei das mindestens eine andere Produkt aus der Gruppe bestehend aus Mundsprays, Nasensprays, Transdermalpflastern, Inhalationsvorrichtungen, Lutschtabletten, Tabletten und Tabak ausgewählt ist.

25. Orale Formulierung nach Anspruch 1 - 22 zur Verwendung bei der Erzielung einer schnellen und/oder anhaltenden und/oder vollständigen Verringerung des Drangs zum Rauchen oder zur Verwendung von Tabak und/oder zur Bereitstellung von Rauchbefriedigung ohne Rauchen.

26. Orale Formulierung nach Anspruch 1 - 22 zur Verwendung bei der Zuführung von Nicotin in beliebiger Form an einen Empfänger.

27. Formulierung nach einem der Ansprüche 1 - 22 zur Verwendung bei der Therapie.

28. Formulierung nach Anspruch 27, wobei sich bei der Therapie um die Behandlung einer Erkrankung aus der Gruppe bestehend aus Tabak- oder Nicotinabhängigkeit, Alzheimer-Krankheit, Morbus Crohn, Parkinson-Krankheit, Tourette-Syndrom, Colitis ulcerosa und Gewichtskontrolle nach dem Aufgeben des Rauchens handelt.

29. Verwendung von Nicotin zur Herstellung einer oralen Formulierung nach einem der Ansprüche 1 - 22 zur Behandlung einer Erkrankung aus der Gruppe bestehend aus Tabak- oder Nicotinabhängigkeit, Alzheimer-Krankheit, Morbus Crohn, Parkinson-Krankheit, Tourette-Syndrom, Colitis ulcerosa und Gewichtskontrolle nach dem Aufgeben des Rauchens.

## Revendications

1. Formulation orale pharmaceutique tamponnée et comprenant de la nicotine, **caractérisée en ce qu'**elle est tamponnée par au moins un acide aminé et/ou un sel de celui-ci, et **en ce qu'**elle comprend un composé d'ajustement du pH lorsque la capacité d'ajustement du pH dudit au moins un acide aminé et/ou du sel de celui-ci est insuffisante ;
**caractérisée en ce que** le au moins un acide aminé et/ou un sel de celui-ci comprend un acide aminé endogène qui est choisi dans le groupe constitué par l'arginine, l'asparagine, l'acide glutamique, la glutamine, l'histidine, l'isoleucine, la leucine, la lysine, la méthionine, l'ornithine, la phénylalanine, la sérine, la thréonine et la valine ; et
**caractérisée en ce que** la formulation orale est dépourvue de tabac.

2. Formulation orale selon la revendication 1, **caractérisée en ce que** le au moins un acide aminé endogène est choisi parmi l'asparagine, l'acide glutamique, la glutamine, l'histidine, l'isoleucine, la leucine, la méthionine, la phénylalanine, la sérine, la thréonine et la valine.

3. Formulation orale selon l'une quelconque des revendications précédentes, étant tamponnée de telle façon que, lors de l'administration orale de la formulation orale à un sujet, le pH de la salive du sujet soit augmenté de 0,2-4 unités de pH, ou de 0, 5-2 unités de pH.

4. Formulation orale selon l'une quelconque des revendications précédentes, étant tamponnée par un acide aminé conjointement avec un ou plusieurs tampons et/ou un ou plusieurs composés d'ajustement du pH choisis dans le groupe constitué par un carbonate y compris le bicarbonate ou le sesquicarbonate, le glycinate, le phosphate, le glycérophosphate ou le citrate d'un métal alcalin, tel que le potassium ou le sodium, ou l'ammonium, y compris le phosphate trisodique, l'hydrogénophosphate disodique, le phosphate tripotassique, l'hydrogénophosphate dipotassique, et l'hydroxyde de calcium, le glycinate de sodium, le trométanol ; et des mélanges de ceux-ci.

5. Formulation orale selon l'une quelconque des revendications 1-4, **caractérisée en ce que** la nicotine sous une forme quelconque est choisie dans le groupe constitué par un sel de nicotine, la forme de base libre de nicotine, un dérivé de nicotine, tel qu'un échangeur de cation de nicotine, un complexe d'inclusion de nicotine ou de la nicotine selon une fixation non covalente quelconque ; la nicotine fixée à des zéolithes ; la nicotine fixée à de la cellulose ou des microsphères d'amidon ; et des mélanges de ceux-ci.

6. Formulation orale selon la revendication 5, **caractérisée en ce que** le complexe d'inclusion de nicotine est un complexe de cyclodextrine, telle que la β-cyclodextrine.

7. Formulation orale selon la revendication 5, **caractérisée en ce que** l'échangeur de cations de nicotine est un échangeur de cations à base de polyacrylate.

8. Formulation orale selon la revendication 5, **caractérisée en ce que** le sel de nicotine est un sel de monotartrate, hydrogénotartrate, citrate, malate et/ou chlorhydrate.

9. Formulation orale selon l'une quelconque des revendications 1-10, **caractérisée en ce que** la nicotine sous une forme quelconque est présente selon une quantité de 0,05-8 mg ou 0,1-6 mg ou 2-5 mg, calculée comme forme de base libre de nicotine par dose unitaire.

10. Formulation orale selon l'une quelconque des revendications 1-9, **caractérisée en ce qu'**éventuellement au moins un ou plusieurs additifs sont choisis dans le groupe constitué par les solvants, les co-solvants, les stabilisants, les conservateurs, les antioxydants, les adoucissants, les épaississants, les liants, les charges, les agents solubilisants, les caoutchoucs, les barrières lipidiques, les agents filmogènes, les émulsifiants, les agents glissants, les lubrifiants, les agents d'enrobage, les véhicules fondants, les édulcorants, les arômes, les substances aromatiques, les agents rafraîchissants, les exhausteurs, les agents colorants, les vitamines, les minéraux, le fluor, les rafraîchisseurs d'haleine, les agents de blanchiment des dents, et des mélanges de ceux-ci.

11. Formulation orale selon l'une quelconque des revendications 1-10, se trouvant sous la forme d'un spray oral, d'une capsule, d'un chewing-gum, d'un comprimé croquable, d'un comprimé, d'un comprimé fondant, d'une tablette, d'un bonbon croquant, d'un bonbon mou, d'un bonbon gélifié, ou d'un film oral.

12. Formulation orale selon l'une quelconque des revendications 1-11, qui est non enrobée.

13. Formulation orale selon l'une quelconque des revendications 1 à 4, **caractérisée en ce que** la formulation orale est un comprimé contenant de la nicotine comprenant du bitartrate de nicotine dihydraté, un acide aminé, du mannitol ou une charge, de la gomme xanthane ou un autre liant, de la crospovidone ou un autre agent délitant, un ou plusieurs arômes, et un ou plusieurs édulcorants artificiels.

14. Formulation orale selon l'une quelconque des revendications 1 à 4, **caractérisée en ce que** la formulation orale est un comprimé orodispersible contenant de la nicotine, comprenant du bitartrate de nicotine dihydraté, de la poudre de cacao ou un(e) autre charge/agent texturant/masqueur de goût, une huile végétale ou un autre véhicule fondant, un acide aminé, du mannitol ou un autre diluant, de la lécithine de soja ou un autre émulsifiant, un agent colorant, des édulcorants artificiels et des agents aromatisants.

15. Formulation orale selon l'une quelconque des revendications 1 à 4, **caractérisée en ce que** la formulation orale est un spray oral contenant de la nicotine, comprenant une base libre de nicotine, de l'éthanol ou un autre solvant, un acide aminé, un poloxamère ou un autre stabilisant, tetracemindinatrium ou un autre stabilisant, et des édulcorants artificiels.

16. Formulation orale selon l'une quelconque des revendications 1 à 4, **caractérisée en ce que** la formulation orale est une capsule molle contenant de la nicotine, comprenant dans le coeur une base libre de nicotine, des triglycérides à chaîne moyenne ou un autre véhicule lipophile, des agents aromatisants, un acide aminé, et un épaississant, dans l'enveloppe interne des matériaux de formation d'une enveloppe hydrophile et dans l'enveloppe externe un matériau de formation et des adoucissants.

17. Formulation orale selon l'une quelconque des revendications 1 à 4, **caractérisée en ce que** la formulation orale est un bonbon dur contenant de la nicotine, comprenant du bitartrate de nicotine dihydraté, de l'isomalt, du maltitol, un acide aminé et un agent aromatisant.

18. Formulation orale selon l'une quelconque des revendications 1 à 4, **caractérisée en ce que** la formulation orale est un film oral contenant de la nicotine, comprenant du bitartrate de nicotine dihydraté, de la gomme xanthane, de la gomme de caroube, un carraghénane, une pectine, un pullulane, un acide aminé, du polysorbate, un agent édulcorant artificiel et un agent aromatisant.

19. Formulation orale selon l'une quelconque des revendications 1 à 4, **caractérisée en ce que** la formulation orale est un bonbon gélifié contenant de la nicotine, comprenant du bitartrate de nicotine dihydraté, de l'isomalt, une pectine, un acide aminé, un agent édulcorant artificiel et un agent aromatisant.

20. Formulation orale selon l'une quelconque des revendications 1 à 4, **caractérisée en ce que** la formulation orale est un bonbon mou contenant de la nicotine, comprenant du bitartrate de nicotine dihydraté, de l'isomalt, de l'huile végétale, un acide aminé, un agent édulcorant et un agent aromatisant.

21. Formulation orale selon l'une quelconque des revendications 1 à 4, **caractérisée en ce que** la formulation orale est un chewing-gum contenant de la nicotine fabriqué par compression directe, comprenant un complexe de résine de nicotine, une base de gomme, un acide aminé, et un ou plusieurs parmi des agents édulcorants, des agents édulcorants artificiels, des agents glissants, des agents aromatisants et des lubrifiants, et éventuellement des agents hydrophobes.

22. Formulation orale selon l'une quelconque des revendications 1 à 4, **caractérisée en ce que** la formulation orale est un chewing-gum contenant de la nicotine fabriqué par malaxage, laminage et découpage, comprenant un complexe de résine de nicotine, une base de gomme, un agent édulcorant, des agents aromatisants et un acide aminé.

23. Système d'administration de nicotine sous une forme quelconque à un sujet, ledit système comprenant une formulation orale selon l'une quelconque des revendications 1-22 et au moins un produit comprenant de la nicotine pour l'obtention d'une réduction de l'envie de fumer ou d'utiliser du tabac.

24. Système selon la revendication 23, **caractérisé en ce que** le au moins un autre produit est choisi dans le groupe constitué par un spray oral, un spray nasal, un patch transdermique, un dispositif d'inhalation, une tablette, un comprimé et du tabac.

25. Formulation orale selon les revendications 1-22, pour une utilisation dans l'obtention d'une réduction rapide et/ou prolongée et/ou complète de l'envie de fumer ou d'utiliser du tabac et/ou pour la fourniture d'une sensation de satisfaction de fumer, sans fumer.

26. Formulation orale selon l'une quelconque des revendications 1-22, pour une utilisation dans l'administration de nicotine sous une forme quelconque à un sujet.

27. Formulation selon l'une quelconque des revendications 1-22, pour une utilisation en thérapie.

28. Formulation selon la revendication 27, **caractérisée en ce que** la thérapie est le traitement d'une maladie choisie dans le groupe constitué par la dépendance au tabac ou à la nicotine, la maladie d'Alzheimer, la maladie de Crohn, la maladie de Parkinson, le syndrome de Tourette, la rectocolite hémorragique, et le contrôle pondéral après l'arrêt du tabac.

29. Utilisation de nicotine pour la production d'une formulation orale selon l'une quelconque des revendications 1-22, pour le traitement d'une maladie choisie dans le groupe constitué par la dépendance au tabac ou à la nicotine, la maladie d'Alzheimer, la maladie de Crohn, la maladie de Parkinson, le syndrome de Tourette, la rectocolite hémorragique, et le contrôle pondéral après l'arrêt du tabac.
